(19) **Europäisches Patentamt**
**European Patent Office**
**Office européen des brevets**

(11) **EP 2 136 208 A2**

(12) **EUROPÄISCHE PATENTANMELDUNG**

(43) Veröffentlichungstag:
**23.12.2009 Patentblatt 2009/52**

(51) Int Cl.:
***G01N 33/00*** *(2006.01)*

(21) Anmeldenummer: **08105826.5**

(22) Anmeldetag: **19.11.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(30) Priorität: **17.06.2008 DE 102008002464**

(71) Anmelder: **Robert Bosch GmbH**
**70442 Stuttgart (DE)**

(72) Erfinder:
- **Grabis, Johannes**
  **71106 Magstadt (DE)**
- **Ochs, Thorsten**
  **71701 Schwieberdingen (DE)**
- **Kamp, Bernhard**
  **71640 Ludwigsburg (DE)**

(54) **Verfahren zur Kompensation der Anströmabhängigkeit des Messsignals eines Gassensors**

(57) Es wird ein Verfahren zur Kompensation der Anströmabhängigkeit des Messsignals eines einer Abgasströmung in einem Abgasrohr einer Brennkraftmaschine ausgesetzten Gassensors angegeben, der eine Messkennlinie ($1/t_{auslös}$ = E * C) für eine festgelegte Referenz-Anströmrichtung ($\alpha_{ref}$) durch das Abgas aufweist.

Bei diesem Verfahren wird mit dem im Betrieb der Bremikraftmaschine einem realen Abgasstrom ausgesetzten, in beliebiger Ausrichtung in das Abgasrohr eingebauten Gassensor mittels Eigendiagnose ein Korrekturfaktor (k) bestimmt und mit dem Korrekturfaktor (k) die Messkennlinie verändert.

FIG. 6

## Beschreibung

Stand der Technik

Die Erfindung geht aus von einem Verfahren zur Kompensation der Anströmabhängigkeit des Messsignals eines einer Abgasströmung in einem Abgasrohr einer Brennkraftmaschine ausgesetzten Gassensors nach dem Oberbegriff des Anspruchs 1.

Gassensoren dieser Art sind z.B. resistive Partikelsensoren, die zur Beladungsprognose von Dieselpartikelfiltern zwecks Regenerationskontrolle eingesetzt werden, um eine hohe Systemsicherheit bei wenigen, effizienten, kraftstoffsparenden Regenerationszyklen des Dieselpartikelfilters zu gewährleisten und kostengünstige Filtermaterialien einsetzen zu können. Resistive Partikelsensoren ordnet sich bei den sammelnden Prinzipien ein, zu denen auch Sensoren zur Messung der Feuchtigkeit oder Sensoren zur Messung der Rußbeladung des Abgases gehören. Solche Sensoren sind beispielsweise aus der DE 101 49 333 A1 sowie aus der DE 10 2004 028 997 A1 bekannt. Der zuletzt genannte Rußsensor weist ein Sensorelement mit einem als Träger dienenden Substrat, z.B. aus einer Aluminiumoxid-Keramik, und mit einer auf den Träger aufgebrachten Widerstandsmessstruktur aus zwei kammartig ineinandergreifenden Messelektroden, auch Interdigitalelektroden genannt, auf, die zur Messung des elektrischen Widerstands einer auf den Messelektroden sich anlagernden, die beiden Messelektroden überdeckenden Rußpartikelschicht dient. Nach Erreichen eines Schwellwertes kann ein sich ändernder Sensorstrom gemessen werden, dessen Änderung eine direkte Information über die pro Zeiteinheit auf der Oberfläche angelagerte Rußmenge darstellt.

Ein solcher Partikelsensor wird in das Abgasrohr einer Brennkraftmaschine so eingesetzt, dass der die Interdigitalelektroden tragende Bereich des Sensorelements, der von einem Schutzrohr umgeben ist, dem Abgasstrom ausgesetzt ist. Ein Teilstrom des Abgases wird dabei durch das Schutzrohr zum Sensorelement hin abgelenkt und überströmt dieses. Aufgrund der konstruktiven Ausführung des Schutzrohrs ist die Durchströmung des Gassensors, also die Überströmung des Sensorelements im Schutzrohr, von der Anströmrichtung des Abgases abhängig. Daher muss der Gassensor immer in einer bestimmten, vorgegebenen Richtung im Abgasrohr verbaut werden.

Offenbarung der Erfindung

Das erfindungsgemäße Verfahren mit den Merkmalen des Anspruchs 1 hat den Vorteil, dass der Gassensor ungerichtet im Abgasrohr verbaut werden kann und die von der Anströmrichtung abhängige Messempfindlichkeit des Gassensors bei Inbetriebnahme der Brennkraftmaschine selbsttätig kompensiert wird und damit keinen Beitrag zum Gesamtfehler des Sensorsignals liefert.

Durch die in den weiteren Ansprüchen aufgeführten Maßnahmen sind vorteilhafte Weiterbildungen und Verbesserungen des im Anspruch 1 angegebenen Verfahrens möglich.

Gemäß einer bevorzugten Ausführungsform des Verfahrens wird bei der Eigendiagnose ein im Abgas strömender Gasmassengesamtstrom und ein davon abgezweigter, den Gassensor durchsetzender Gasmassenteilstrom bestimmt. Aus einem abgespeicherten, bekannten Zusammenhang der Abhängigkeit des Gasmassenteilstroms vom Gasmassengesamtstrom bei Anströmung des Gassensors durch den Gasmassengesamtstrom in einer Referenz-Anströmrichtung wird zu dem bei Eigendiagnose momentan bestimmten Gasmassengesamtstrom derjenige Gasmassenteilstrom entnommen, der bei Referenz-Anströmrichtung den Gassensor durchsetzen würde. Dieser Gasmassenteilstrom und der bei Eigendiagnose bestimmte, momentane Gasmassenteilstrom, der den ungerichteten, also nicht in Referenzlage, eingebauten Gassensor durchsetzt, werden ins Verhältnis gesetzt und daraus ein Korrekturfaktor abgeleitet. Während der Gasmassengesamtstrom in jedem Betriebspunkt der Brennkraftmaschine bekannt ist, da er für die Steuerung der Kraftstoffeinspritzung benötigt wird und mittels eines Luftmassenmessers und der eingespritzten Kraftstoffmenge bestimmt wird, wird zur Bestimmung des Gasmassenteilstroms eine Hilfsgröße verwendet, die ein Maß für die Abkühlung des Sensorelements im Gassensor durch den den Gassensor durchsetzenden Gasmassenteilstrom und damit proportional zur Größe des Gasmassenteilstroms ist. Eine solche Hilfsgröße ist die einem im Gassensor integrierten Heizer zugeführte Heizleistung, die abhängig von dem Gasmassengesamtstrom und der Abgastemperatur erforderlich ist, um den Gassensor bzw. dessen Sensorelement auf eine vorbestimmte Temperatur zu erwärmen. Da die benötigte Heizleistung abhängt von der durch den Gasmassenteilstrom bewirkten Abkühlung des Sensorelements, kann der Korrekturfaktor unmittelbar aus dem Vergleich der bei Anströmung des Gassensors in Referenz-Anströmrichtung gemessenen Heizleistung und der während der Eigendiagnose bei unbekannter Anströmrichtung gemessenen Heizleistung ermittelt werden.

Kurze Beschreibung der Zeichnungen

Das erfindungsgemäße Verfahren ist anhand eines in der Zeichnung dargestellten Ausführungsbeispiels in der nachfolgen Beschreibung näher erläutert. Es zeigen:

Fig. 1   ausschnittweise eine Brennkraftmaschine mit einem Kraftstoffversorgungssystem und einem Abgastrakt, schematisiert dargestellt,

Fig. 2   ausschnittweise einen Längsschnitt eines Gassensors im Abgastrakt der Brennkraftmaschine in Fig. 1,

Fig. 3    ausschnittweise eine Draufsicht eines Sensorelements des Gassensors in Richtung Pfeil III in Fig. 2,

Fig. 4    eine Darstellung eines schematisierten Schnitts gemäß Linie IV-IV in Fig. 2 nach Einbau des Gassensors in ein Abgasrohr des Abgastrakts der Brennkraftmaschine in Fig. 1 in drei verschiedenen Ausrichtungen bezüglich der Rohrachse,

Fig. 5    ein Diagramm der Messempfindlichkeit E des Gassensors in Abhängigkeit von seiner Anströmrichtung $\alpha$ durch das Abgas im Abgasrohr der Fig. 4,

Fig. 6    eine in einem Steuergerät der Brennkraftmaschine abgespeicherte Messkennlinie des Gassensors, schematisiert dargestellt,

Fig. 7    ein ebenfalls im Steuergerät der Brennkraftmaschine abgespeichertes Kennlinienfeld der Heizleistung eines im Gassensor integrierten elektrischen Heizers als Funktion des Gasmassengesamtstroms im Abgasrohr des Abgastrakts und der Abgastemperatur, bezogen auf eine vorgegebene Referenz-Anströmrichtung des Gassensors, schematisiert dargestellt.

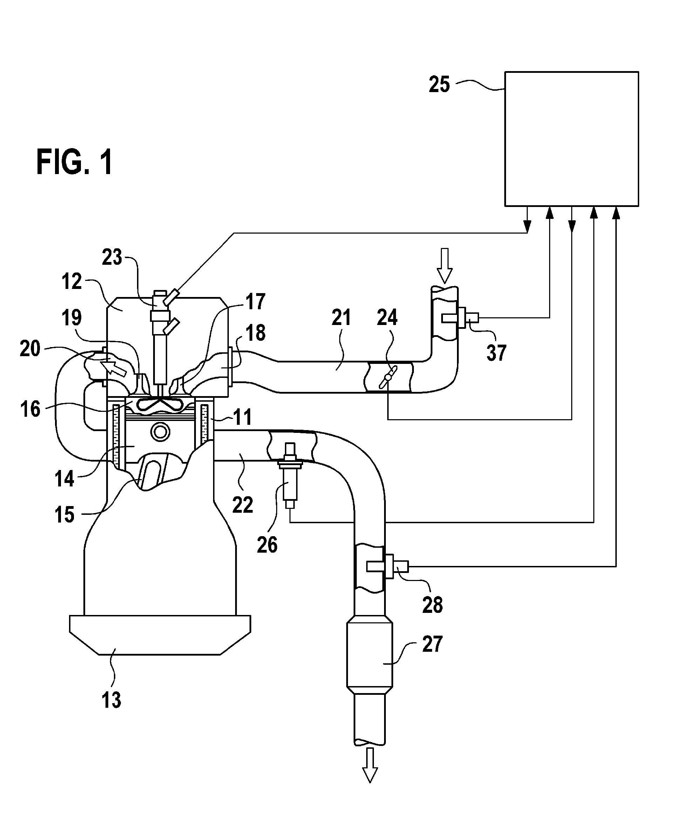

Die in Fig. 1 schematisiert dargestellte Brennkraftmaschine weist in bekannter Weise mehrere hintereinander angeordnete Verbrennungszylinder 11 auf, von denen in Fig. 1 ein Verbrennungszylinder 11 zu sehen ist, die stirnseitig von einem Zylinderkopf 12 abgedeckt sind. In einem Kurbelwellengehäuse 13 ist eine nicht dargestellte Kurbelwelle gelagert. Mit der Kurbelwelle sind in den Verbrennungszylindern 11 axial geführte Hubkolben 14 über jeweils ein Pleuel 15 verbunden. In jedem Verbrennungszylinder 11 ist zwischen dem Zylinderkopf 12 und dem Hubkolben 14 ein Verbrennungsraum 16 eingeschlossen, der über mindestens ein Einlassventil 17 mit einem im Zylinderkopf 12 ausgebildetem Einlasskanal 18 und über mindestens ein Auslassventil 19 mit einem im Zylinderkopf 12 ausgebildeten Auslasskanal 20 in Verbindung steht. An den mindestens einen Einlasskanal 18 ist ein Luftansaugrohr 21 eines Kraftstoffversorgungssystems und an den mindestens einen Auslasskanal 20 ein Abgasrohr 22 eines Abgastrakts angeschlossen. Die Brennkraftmaschine ist mit einer Kraftstoffdirekteinspritzung ausgestattet, bei der mindestens ein Kraftstoffeinspritzventil 23 unter Hochdruck stehenden Kraftstoff direkt in den Verbrennungsraum 16 eines Verbrennungszylinders 11 einspritzt. Die zur Kraftstoffverbrennung dem Verbrennungsraum 16 zugeführte Verbrennungsluft wird mittels einer Regelklappe 24 gesteuert. Die Zumessung der Kraftstoffeinspritzmenge und der Luftmenge wird abhängig vom Betriebspunkt der Brennkraftmaschine von einem Steuergerät 25 gesteuert. Dem Steuergerät 25 sind als Betriebsgrößen das Ausgangssignal eines im Luftansaugrohr 21 angeordneten Luftmassenmessers 37 und das Ausgangssignal einer im Abgasrohr 22 angeordneten Lambdasonde 26 zugeführt.

Zur Reduzierung des Partikelausstoßes der Brennkraftmaschine über das Abgas ist im Abgasrohr 22 ein Partikelfilter 27 und stromaufwärts des Partikelfilters 27 ein resistiver Partikelsensor 28 angeordnet. Mit dem Partikelsensor 28 wird die Partikelbeladung des Abgases überwacht und eine Beladungsprognose für den Partikelfilter 27 zur Regenerationskontrolle vorgenommen. Bei einem anderen Einsatzprofil des Partikelsensors 28 ist dieser stromabwärts des Pertikelfilters 27 angeordnet und dient zur Überwachung des Partikelausstoßes während des Fahrbetriebs.

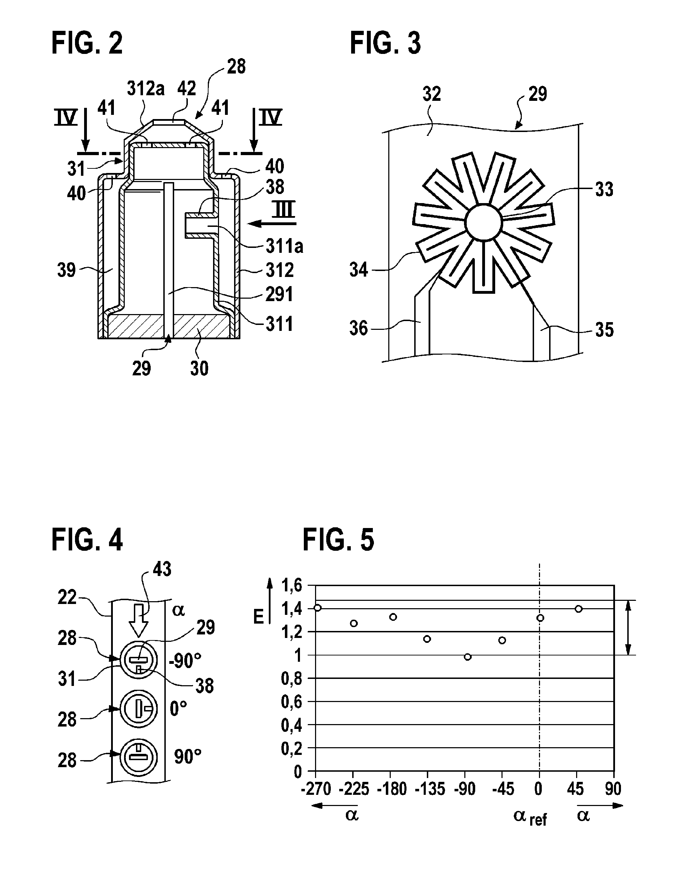

Resistive Partikelsensoren 28, die nach dem sammelnden Prinzip arbeiten, wie z.B. Rußpartikelsensoren, sind bekannt und in Aufbau und Funktionsweise beispielsweise in der EP 1 624 166 A1 oder DE 10 2004 028 997 A1 beschrieben. Ein bevorzugtes Ausführungsbeispiel für den Partikelsensor 28, der beispielhaft für einen allgemeinen, der Abgasströmung im Abgasrohr ausgesetzten Gassensor herangezogen ist, weist ein Sensorelement 29 auf, das in einem Sensorgehäuse 30 aufgenommen ist und mit einem gassensitiven Endabschnitt 291 aus dem Sensorgehäuse 30 herausragt (Fig. 2). Der Endabschnitt 291 ist von einem Doppelschutzrohr 31 überdeckt, das am Sensorgehäuse 31 festgelegt ist. Das Sensorelement 29 weist einen Keramikträger 32 für ein sog. Interdigitalelektrodensystem auf. Dieses besteht aus zwei kammartig ineinandergreifenden Messelektroden 33, 34, die auf der einen Großfläche des Sensorelements 29 im Endabschnitt 291 angeordnet sind. Im besachriebenen Ausführungsbeispiel sind beide Messelektroden 33, 34 radial ausgerichtet und jeweils mit einer Anschlussleiterbahn 35 bzw. 36 verbunden. Die Messelektroden können auch parallel zueinander ausgerichtet sein und kammartig ineinandergreifen, wie dies z.B. in der DE 10 2004 028 997 A1 beschrieben ist. Außerdem ist im Sensorelement 29 ein elektrischer Heizer (nicht dargestellt) in Form einer als Mäander ausgebildeten Widerstandsbahn integriert, mit dem das Sensorelement 29 auf eine vorbestimmte Temperatur aufheizbar ist. Das den Endabschnitt 291 überdeckende Doppelschutzrohr 31 ist so ausgeführt, dass durch einen das Doppelschutzrohr 31 durchströmenden, von Abgasstrom im Abgasrohr 22 abgezweigten Abgasteilstrom eine sog. Staupunktanströmung im Zentrum des Interdigitalelektrodensystems hervorgerufen wird und das Abgas dann radial über die Messelektroden 33, 34 abströmt. Um diese Staupunktanströmung zu realisieren weist das innere Schutzrohr 311 des aus kappenförmigem inneren Schutzrohr 311 und kappenförmigem äußeren Schutzrohr 312 bestehenden Doppelschutzrohrs 31 ein auf das Zentrum des Interdigitalelektrodensystems weisendes Anströmröhrchen 38 auf, das eine Gaseintrittsöffnung 311 a im Rohrmantel des inneren Schutzrohrs 311 umschließt und radial vom Rohrmantel des inneren Schutzrohrs 311 hin zum Sensorelement 29 absteht. Das kappenförmige äußere Schutzrohr 312, das mit einem den Endabschnitt des inneren Schutzrohrs 311 aufnehmenden, tiefgezogenen Dom 312a im Kappenboden versehen ist, umschließt das innere Schutzrohr 311 mit Radialabstand, so dass sich zwischen den beiden Schutzrohren 311, 312 ein Ringraum 39 ausbildet.

Nach Einbau des Partikelsensors 28 in das Abgasrohr 22 ragt das Doppelschutzrohr 31 frei in das Abgasrohr 22 hinein und ist dem im Abgasrohr 22 strömenden Gasmassengesamtstrom ausgesetzt, wobei ein Gasmassenteilstrom das Doppelschutzrohr 31 durchsetzt und das Sensorelement 29 beaufschlagt. Das Abgas tritt dabei über Gaseintrittslöcher 40 im Kappenboden des äußeren Schutzrohrs 312 in den Ringraum 39 ein und erzeugt über das Anströmröhrchen 38 die beschriebene Staupunktanströmung des Sensorelements 29. Das vom Sensorelement 29 abströmende Abgas tritt über Gasdurchtrittslöcher 41 im Kappenboden des inneren Schutzrohrs 311 und durch ein zentrales Gasaustrittsloch 42 im Dom 312a des äußeren Schutzrohrs 312 wieder aus dem Doppelschutzrohr 31 aus. Die Messempfindlichkeit des Partikelsensors 28 hängt davon ab, welcher Anteil am Gasmassengesamtstrom den Gassensor als Gasmassenteilstrom durchsetzt. Dieser Anteil ist abhängig von der Richtung der Anströmung des Partikelsensors 28 durch das Abgas im Abgasrohr 22 und damit von der Einbaulage des Partikelsensors 28 im Abgasrohr 22. Im Diagramm der Fig. 5 ist beispielhaft die Messempfindlichkeit E in Abhängigkeit von der Anströmrichtung $\alpha$ eingetragen. Für die Anströmrichtung $\alpha$ des Partikelsensors 28 durch das Abgas sind in Fig. 4 drei Beispiele dargestellt, wobei symbolhaft das Sensorelement 29, das Doppelschutzrohr 31 und das Anstromröhrchen 38 im Schnitt dargestellt sind. Da die Strömungsrichtung des Abgases im Abgasrohr 22 festgelegt ist (Pfeil 43 in Fig. 4), ist die Anströmrichtung $\alpha$ gleichzusetzen mit einer Drehwinkellage des Partikelsensors 28 um seine Längsachse. Dabei ist eine Anströmrichtung als Referenz mit $\alpha_{ref}$ = 0° festgelegt. Wird die Anströmrichtung $\alpha$ gegenüber der Referenz-Anströmrichtung $\alpha_{ref}$ verändert, z.B. um -90° oder +90°, so ändert sich die Messempfindlichkeit E des Partikelsensors, wie dies in Fig. 5 dargestellt ist.

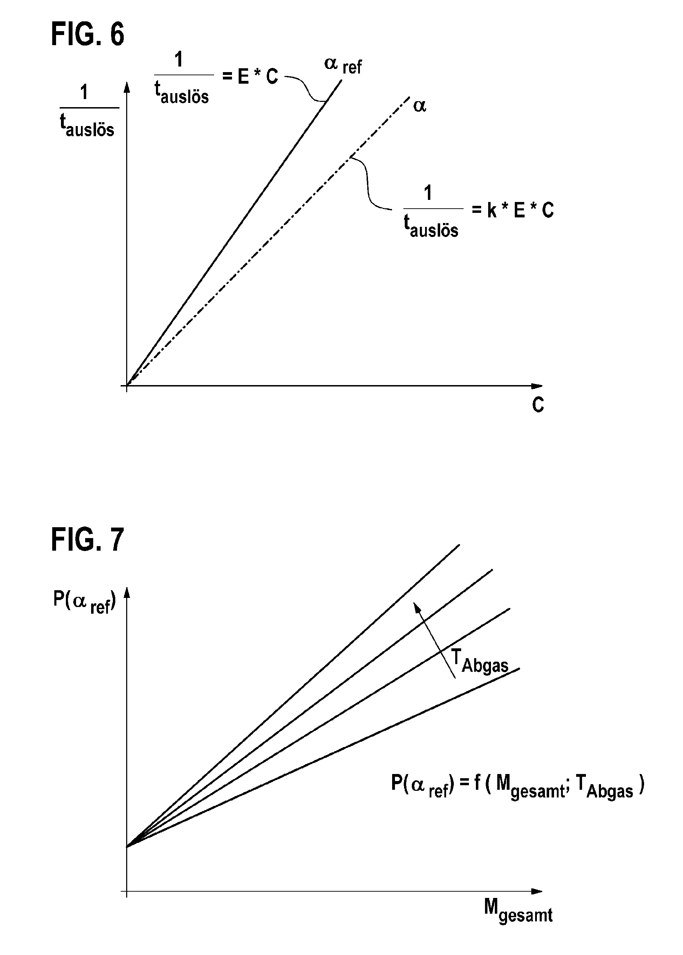

Bei der Durchströmung des Partikelsensors 28 lagern sich die elektrisch leitfähigen Partikel auf der Oberfläche des Sensorelements 29 an. Bei ausreichender Beladung mit Partikeln führt dies zu einem ansteigenden Stromfluss zwischen den Messelektroden 33, 34, der mit dem Ausmaß der Beladung korreliert. Die Zeit zwischen Beginn der Partikelanlagerung und Überschreiten einer bestimmten Stromschwelle wird als Auslösezeit bezeichnet. Der Zusammenhang zwischen der Partikelkonzentration im Abgas und der Auslösezeit wird als Messkennlinie des Partikelsensors 28 bei einer Anströmrichtung des Partikelsensors 28 durch das Abgas, die der Referenz-Anströmrichtung $\alpha_{ref}$ entspricht, im Steuergerät 25 abgelegt. Dabei wird der Kehrwert der Auslösezeit $t_{auslös}$ als Funktion der Partikelkonzentration C gemäß

$$1/t_{auslös} = E * C$$

vorgegeben, wobei die Empfindlichkeit E durch die Menge des am Sensorelement 29 vorbeiströmenden Abgases und durch die Anlagerungsrate der Partikel aus dem Abgas auf dem Sensorelement 29 gegeben ist. In Fig. 6 ist beispielhaft eine solche Messkennlinie ausgezogen dargestellt.

Die Gültigkeit dieser im Steuergerät abgespeicherten Messkennlinie setzt voraus, dass der Partikelsensor 28 so in das Abgasrohr 22 eingesetzt ist, dass die Anströmrichtung $\alpha_{ref}$ = 0 eingehalten wird. Dies entspricht beispielsweise der in Fig. 4 dargestellten mittleren Einbaulage des Partikelsensors 28, bei der das Anströmröhrchen 38 im Doppelschutzrohr 31 quer zu dem im Abgasrohr 22 fließenden Abgasstrom, der durch Pfeil 43 in Fig. 4 gekennzeichnet ist, ausgerichtet ist.

Um eine solche Einbauvorschrift für den Partikelsensor 28 in das Abgasrohr 22 zu umgehen und eine von der Einbaulage des Partikelsensors 28 unabhängige Messgenauigkeit des Partikelsensors 28 zu erhalten, wird nach beliebigem Einbau des Partikelsensors 28 in das Abgasrohr 22 mit dem im Betrieb der Brennkraftmaschine dem realen Abgasstrom im Abgasrohr 22 ausgesetzten Partikelsensor 28 mittels Eigendiagnose ein Korrekturfaktor k bestimmt und mit dem Korrekturfaktur k die Messkennlinie entsprechend verändert. Die Veränderung erfolgt in der Weise, dass die Messempfindlichkeit E des Gassensors, also die Steigung der Messkennlinie in Fig. 6, mit dem Korrekturfaktor k multipliziert wird, so dass für den nunmehr in das Abgasrohr 22 eingebauten Partikelsensor die Kennlinie

$$1/t_{auslös} = k * E * C$$

gilt, wie diese in Fig. 6 strichpunktiert eingezeichnet ist. Im Grundsatz wird bei der Eigendiagnose der im Abgasrohr 22 strömende Gasmassengesamtstrom und der davon abgezweigte, den Partikelsensor 28, genauer das Doppelschutzrohr 31 mit Sensorelement 29, durchsetzende Gasmassenteilstrom bestimmt und der Korrekturfaktor k ins umgekehrte Verhältnis zu einem bekannten Gasmassenteilstrom gesetzt, der bei wie bestimmt gleichem Gassmassengesamtstrom im Abgasrohr 22 den Partikelsensor 28 bei dessen Anströmung in Referenz-Anströmrichtung $\alpha_{ref}$

durchsetzt. Der Gasmassengesamtstrom im Abgas wird durch Messen der pro Zeiteinheit der Brennkraftmaschine zugeführten Luft- und Krafstoffmengen bestimmt. Diese Daten sind im Steuergerät 25 bereits vorhanden, da sie zur Steuerung der Kraftstoffeinspritzung erforderlich sind. Der den Partikelsensor 28 durchströmende Gasmassenteilstrom kann prinzipiell aus dem Abtransport der Wärme vom Sensorelement 29 bestimmt werden. Hierzu wird als Hilfsgröße eine Heizleistung P gemessen, die abhängig von dem Gasmassengesamtstrom $M_{gesamt}$ und der Abgastemperatur $T_{Abgas}$ dem im Partikelsensor integrierten elektrischen Heizer zugeführt werden muss, damit der Partikelsensor 28, genauer das Sensorelement 29, sich auf eine vorbestimmte Temperatur erwärmt. Diese Heizleistung ist ein Maß für den durch das Doppelschutzrohr 31 strömenden Gasmassenteilstrom.

Um den bekannten Gasmassensteilstrom, der in Referenz-Anströmrichtung $\alpha_{ref}$ durch das Abgas den Partikelsensor 28 durchsetzt, zu bestimmen, wird in einer Applikations- oder Abstimmungsphase für einen bestimmten Brennkraftmaschinentyp der Partikelsensor 28 so in das Abgasrohr 22 eingebaut, dass die Anströmung des Partikelsensors 28 durch das Abgas in Referenz-Anströmrichtung $\alpha_{ref}$ erfolgt. Im anschließenden Betrieb der Brennkraftmaschine wird zu verschiedenen Betriebspunkten einerseits der durch das Abgasrohr 22 strömende Gasmassengesamtstrom $M_{gesamt}$ sowie die Abgastemperatur $T_{Abgas}$ bestimmt und die Heizleistung $P(\alpha_{ref})$ des elektrischen Heizers bestimmt, die dem Partikelsensor 28 zugeführt wird, um diesen auf eine vorbestimmte Temperatur zu erwärmen. Diese als Funktion des Gasmassengesamtstroms und der Abgastemperatur bestimmte Heizleistung

$$P (\alpha_{ref}) = f (M_{gesamt}; T_{Abgas})$$

wird als Kennlinienfeld im Steuergerät 25 abgespeichert, wie dies in Fig. 7 schematisiert dargestellt ist.

Bei der individuellen Brennkraftmaschine dieses in der Applikationsphase so vermessenen Brennkraftmaschinentyps wird nach beliebigem, ungerichtetem Einbau des Partikelsensors 28 in das Abgasrohr 22 bei der unmittelbar nach Inbetriebnahme der individuellen Brennkraftmaschine erfolgenden Eigendiagnose zur Bestimmung des Korrekturfaktors k für die Messkennlinie - wie bereits beschrieben - einerseits der Gasmassengesamtstrom $M_{gesamt}$ sowie die Abgastemperatur $T_{Abgas}$ des elektrischen Heizers und andererseits die Heizleistung $P(\alpha)$ des elektrischen Heizers für das Aufheizen des Sensorelements 29 auf die vorbestimmte Temperatur bestimmt. Mit dem bestimmten Gasmassengesamtstrom $M_{gesamt}$ und der bestimmten Abgastemperatur $T_{Abgas}$ wird aus dem Kennlinienfeld gemäß Fig. 7

die Heizleistung P ($\alpha_{ref}$) entnommen und ins Verhältnis zu der bestimmten Heizleistung P ($\alpha$) gesetzt. Der Korrekturfaktor k ist proportional zu diesem Quotienten P ($\alpha_{ref}$) / P($\alpha$).

Mit dem Korrekturfaktor k wird nunmehr, wie beschrieben, die Messkennlinie gemäß Fig. 6 korrigiert, wobei die die Empfindlichkeit des Gassensors repräsentierende Steilheit der Messkennlinie bei einer gegenüber der Heizleistung P ($\alpha_{ref}$) bei Referenz-Anströmrichtung gemessenen größeren Heizleistung P ($\alpha$) nach unten (k<1) und bei einer kleineren Heizleitung P($\alpha$) nach oben korrigiert wird (k>1). Die durch Eigendiagnose bestimmte gültige Messkennlinie ist somit durch die Funktion

$$1/t_{auslös} = k * E * C$$

festgelegt ist, wie sie in Fig. 6 strichpunktiert dargestellt ist. Bei Verwendung dieser Messkennlinie zur Ermittlung der Partikelbeladung des Abgases ist die Messungenauigkeit des Partikelsensors 28, die durch die von der Referenz-Anströmrichtung $\alpha_{ref}$ abweichende Anströmrichtung $\alpha$ des Partikelsensors 28 durch das Abgas bedingt ist, kompensiert.

Zur Bestimmung der erforderlichen Heizleistung P wird die an dem elektrischen Heizer liegende Spannung gemessen und aus der gemessenen Spannung und dem bekannten Widerstand des elektrischen Heizers die Heizleistung berechnet.

Die festgelegte Temperatur, auf die der Partikelsensor 28 mit der gemessenen Heizleistung P aufgeheizt wird, wird mittels eines in dem Partikelsensor 28 integrierten Messmäanders bestimmt. Die Heizerspannung wird so geregelt, dass sich ein fester Widerstand des Messmäanders einstellt. Dieser Widerstandswert entspricht aufgrund der temperaturabhängigen Leitfähigkeit des Metalls des Messmäanders einer bestimmten Temperatur. Alternativ kann die Temperaturschwelle für die Erwärmung des Partikelsensors 28 auch indirekt über die Leitfähigkeit des interdigitalen Elektrodensystems des Sensorelements 29 bestimmt werden. Hierzu wird ein vorher festgelegter Wert für die Leitfähigkeit der beiden Messelektroden 33, 34 bei einer festgelegten Heizleistung und Abgastemperatur mit der tatsächlich erreichten Leitfähigkeit verglichen. Die elektrische Leitfähigkeit ist ein Maß für die Temperatur, da der Keramikträger 32 bei hoher Temperatur elektrisch leitfähig wird.

Das vorstehend beschrieben Verfahren der Eigendiagnose bei ungerichtetem Einbau des Partikelsensors 28 ist auch für alle Gassensoren anwendbar, deren Messsignal vom Abgasdurchsatz durch das Abgasrohr abhängig ist, deren Messsignal also direkt mit der am Sensorelement vorbeiströmenden Gasmenge korreliert. Hierzu gehören alle nach einem sammelnden Prinzip arbeitende Gassensoren, wie resistive Partikelsensoren, z.B. Ruß- oder Feuchtigkeitssensoren, aber auch solche, die andere Abgasbestandteile, wie Kohlenwasserstoffe, Stickoxide, Ammoniak u. dgl., sammeln und daraus ein Messsignal ableiten.

**Patentansprüche**

1. Verfahren zur Kompensation der Anströmabhängigkeit des Messsignals eines einer Abgasströmung in einem Abgasrohr (22) einer Brennkraftmaschine ausgesetzten Gassensors, der eine Messkennlinie für eine festgelegte Referenz-Anströmrichtung ($\alpha_{ref}$) durch das Abgas aufweist, **dadurch gekennzeichnet, dass** mit dem im Betrieb der Brennkraftmaschine einem realen Abgasstrom ausgesetzten Gassensor mittels Eigendiagnose ein Korrekturfaktor (k) bestimmt und mit dem Korrekturfaktor (k) die Messkennlinie verändert wird.

2. Verfahren nach Anspruch 1, **dadurch gekennzeichnet, dass** bei der Eigendiagnose ein im Abgasrohr (22) strömender Gasmassengesamtstrom ($M_{gesamt}$) und ein davon abgezweigter, den Gassensor durchsetzender Gasmassenteilstrom bestimmt wird und dass zur Bestimmung des Korrekturfaktors (k) der bestimmte Gasmassenteilstrom so ins Verhältnis zu einem abgespeicherten, bekannten Gasmassenteilstrom, der bei wie bestimmt gleichem Gasmassengesamtstrom ($M_{gesamt}$) im Abgasrohr (22) den Gassensor bei Anströmung in Referenz-Anströmrichtung ($\alpha_{ref}$) durchsetzt, gesetzt wird, dass die Steigung des Messkennlinie nach unten korrigiert wird, wenn der bestimmte Gasmassenteilstrom größer und nach oben korrigiert wird, wenn der bestimmte Gasmessenteilstrom kleiner als der abgespeicherte, bekannte Gasmassenteilstrom ist.

3. Verfahren nach Anspruch 2, **dadurch gekennzeichnet, dass** als zum Gasmassenteilstrom proportionale Hilfsgröße eine Heizleistung (P) bestimmt wird, die abhängig von dem Gasmassengesamtstrom ($M_{gesamt}$) und der Abgastemperatur ($T_{Abgas}$) einem im Gassensor integrierten, elektrischen Heizer zugeführt werden muss, um den Gassensor auf eine vorbestimmte Temperatur zu erwärmen.

4. Verfahren nach Anspruch 3, **dadurch gekennzeichnet, dass** die bei Referenz-Anströmrichtung ($\alpha_{ref}$) des Gassensors für ausgewählte Betriebszustände der Brennkraftmaschine gemessene Referenz-Heizleistung ($P(\alpha_{ref})$) als Funktion des Gasmassengesamtstroms ($M_{gesamt}$) und der Abgastemperatur ($T_{Abgas}$) abgespeichert wird, dass aus der abgespeicherten Funktion der Referenz-Heizleistung ($P(\alpha_{ref})$) für bei der Eigendiagnose bestimmten Gasmassengesamtstrom ($M_{gesamt}$) und Abgastemperatur ($T_{Abgas}$) die Referenz-Heizleistung ($P(\alpha_{ref})$) entnommen wird und dass der Korrekturfaktor (k) aus dem Vergleich der Referenz-Heizleistung ($P(\alpha_{ref})$) mit der bei Eigendiagnose bestimmten Heizleistung ($P(\alpha)$) ermittelt wird.

5. Verfahren nach Anspruch 4, **dadurch gekennzeichnet, dass** der Korrekturfaktor als eine dem Quotienten aus der Referenz-Heizleistung ($P(\alpha_{ref})$) und der bei Eigendiagnose bestimmten Heizleistung $P(\alpha)$) proportionale Größe berechnet wird.

6. Verfahren nach einem der Ansprüche 1 bis 5, **dadurch gekennzeichnet, dass** der Gasmassengesamtstrom ($M_{gesamt}$) im Abgasrohr (22) durch Messen der pro Zeiteinheit der Brennkraftmaschine zugeführten Luft- und Kraftstoffmengen bestimmt wird.

7. Verfahren nach einem der Ansprüche 3 bis 6, **dadurch gekennzeichnet, dass** die an dem elektrischen Heizer anliegende Spannung gemessen und aus der gemessenen Spannung und dem bekannten Heizwiderstand die Heizleistung (P) berechnet wird.

8. Verfahren nach einem der Ansprüche 3 bis 7, **dadurch gekennzeichnet, dass** die durch die Heizleistung (P) zu erreichende vorbestimmte Temperatur des Gassensors mittels eines im Gassensor integrierten Messmäanders bestimmt wird.

**FIG. 1**

FIG. 2

FIG. 3

FIG. 4

FIG. 5

## FIG. 6

$$\frac{1}{t_{auslös}} = E * C$$

$\alpha_{ref}$

$\alpha$

$$\frac{1}{t_{auslös}} = k * E * C$$

$\frac{1}{t_{auslös}}$

C

## FIG. 7

$P(\alpha_{ref})$

$T_{Abgas}$

$$P(\alpha_{ref}) = f(M_{gesamt}; T_{Abgas})$$

$M_{gesamt}$

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10149333 A1
- DE 102004028997 A1
- EP 1624166 A1